# EUROPÄISCHE PATENTSCHRIFT

(11) **EP 0 555 179 B1**
(45) Veröffentlichungstag und Bekanntmachung des Hinweises auf die Patenterteilung: **15.09.1999**
(21) Anmeldenummer: 93810054.2
(22) Anmeldetag: 27.01.1993
(51) Int. Cl.: C09B 29/085, C09B 31/043, C07C 229/16, C07C 233/43, C07C 271/28, C07C 275/40, C07C 311/05

(54) **Azofarbstoffe**
Azo dyestuffs
Colorants azoiques

(30) Priorität: 03.02.1992 CH 30092
(43) Veröffentlichungstag der Anmeldung: 11.08.1993
(73) Patentinhaber: Ciba Specialty Chemicals Holding Inc., 4057 Basel (CH)
(72) Erfinder: Trottmann, Martin, Dr., CH-4106 Therwil (CH)

(56) Entgegenhaltungen:
- DE-A- 2 612 792
- FR-A- 1 377 496
- US-A- 2 373 700
- CHEMICAL ABSTRACTS, vol. 94, no. 10, März 1981, Columbus, Ohio, US; abstract no. 67273a, 'azo dyes' Seite 87 & JP-A-55 116 754 (MITSUI TOATSU CHEMICALS, INC.)
- CHEMICAL ABSTRACTS, vol. 106, no. 16, April 1987, Columbus, Ohio, US; abstract no. 121389z, 'azo dye for synthetic and semi-synthetic fibers' Seite 86 & JP-A-61 160 490 (MITSUI TOATSU CHEMICALS, INC.)
- CHEMICAL ABSTRACTS, vol. 100, no. 10, März 1984, Columbus, Ohio, US; abstract no. 69851d, 'disperse monoazo dyes' Seite 80 & JP-A-58 160 355 (SUMITOMO CHEMICAL COMPANY, LTD.)

## Beschreibung

Gegenstand der vorliegenden Erfindung sind Dispersionsfarbstoffe, Verfahren zu deren Herstellung sowie ihre Verwendung zum Färben von Textilmaterialien.

Dispersionsfarbstoffe, d.h. Farbstoffe, welche keine wasserlöslichmachenden Gruppen enthalten, sind seit langem bekannt und werden zum Färben von hydrophobem Textilmaterial verwendet. Vielfach sind die erhaltenen Färbungen jedoch nicht ausreichend thermomigrierecht. Dieses Problem tritt vor allem bei roten bis blauen Nuancen auf.

Zur Behebung dieses Mangels wurden schon spezielle Farbstoffe entwickelt, deren Diffusionsvermögen infolge ihrer molekularen Grösse und/oder Sperrigkeit möglichst niedrig ist. Dies erschwert jedoch oft die Anwendbarkeit solcher Farbstoffe, da man mit ihnen nicht oder nur schlecht nach dem Ausziehverfahren färben kann und diese Farbstoffe sogar im Thermosol-Verfahren meistens unerwünscht hohe Fixiertemperaturen erfordern.

So sind z.B aus US-A-2,373,700 Azofarbstoffe bekannt, welche als Kupplungskomponente ein N,N-disubstituierten Anilin beinhalten.
DE-A-26 12 792 offenbart Thiophen-Azofarbstoffe, welche als Kupplungskomponente 3-Methyl-N,N-di-(methoxycarbonylmethyl)anilin enthalten.
JP-A-55116754 offenbart Azofarbstoffe welche als Kupplungskomponente ein gegebenenfalls weiter substituiertes 3-Alkylcarbonylamino-N,N-di-(methoxycarbonylmethyl)anilin und als Azokomponente ein spezifisch substituiertes p-Nitroanilin enthalten.
JP-A-61160490 beschreibt Benzothiazol-Azofarbstoffe mit einer 3-Acetamino-N,N-di-(methoxycarbonylmethyl)anilin-Kupplungskomponente.

Gegenstand der vorliegenden Erfindung sind nun Dispersionsfarbstoffe, welche sehr thermomigrierechte Färbungen ergeben, aber trotzdem sowohl im Auszieh- und Thermosolverfahren als auch im Textildruck ein gutes Aufbauvermögen besitzen. Die Farbstoffe sind auch für den Aetzdruck geeignet.

Die erfindungsgemässen Farbstoffe entsprechen der Formel 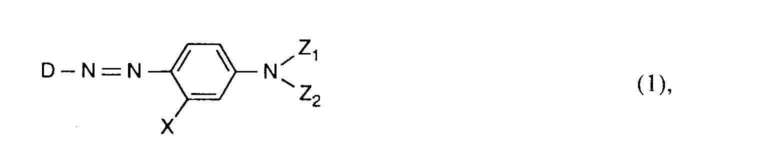 worin
D den Rest einer Diazokomponente der Formel 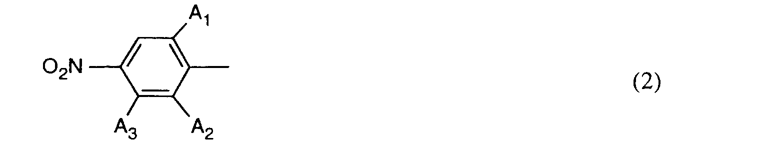 Z₁ und Z₂ einen Rest der Formel 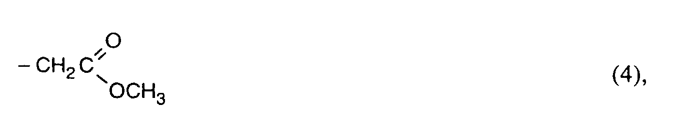
X C₁-C₂-Alkyl, C₁-C₄-Alkylcarbonylamino, C₁-C₄-Alkoxycarbonylamino, C₁-C₄-Alkoxy-C₁-C₄-Alkylcarbonylamino oder
   C₁-C₄-Alkoxy-C₂-C₄-Alkoxycarbonylamino,
A₁ Wasserstoff, Halogen, SO₂R₃, CF₃, NO₂ oder CN,
A₂ Wasserstoff, Halogen oder CN,
A₃ Wasserstoff oder Halogen ist, und
R₃ C₁-C₆-Alkyl sind.

Unter Alkylresten sind erfindungsgemäss generell geradkettige, verzweigte oder cyclische Alkylgruppen zu verstehen. Es handelt sich z.B. um Methyl, Ethyl, Propyl, i-Propyl, Butyl, i-Butyl, tert.-Butyl, Amyl, tert.-Amyl(1,1-Dimethylpropyl), Hexyl, 1-Methylpentyl, sowie die dazugehörenden Isomeren. Die Alkylreste enthalten vorzugsweise 1 bis 4 C-Atome, sofern nicht spezifisch angegeben.

Geeignete Alkoxyreste sind vorzugsweise solche mit 1-4 C-Atomen z.B. Methoxy, Ethoxy, Propoxy, iso-Propoxy, n-Butoxy, iso-Butoxy oder tert.-Butoxy.

Unter Halogen ist in dieser Anmeldung generell Fluor, Brom oder Jod und vor allem Chlor zu verstehen.

In besonders wertvollen Farbstoffen der Formel (1) stellt D einen Rest der Formel (2) worin A₁ vorzugsweise Chlor, Brom, Cyan, Nitro, CF₃ oder Wasserstoff, A₂ Wasserstoff, Chlor, Brom oder Cyan und A₃ Wasserstoff bedeuten, dar. Unter diesen sind diejenigen von besonderem Interesse, bei denen A₁ Chlor, Cyan oder Nitro und A₂ Wasserstoff, Chlor, Brom oder Cyan bedeuten.

Wegen ihrer guten färberischen Eigenschaften sind Farbstoffe der Formel 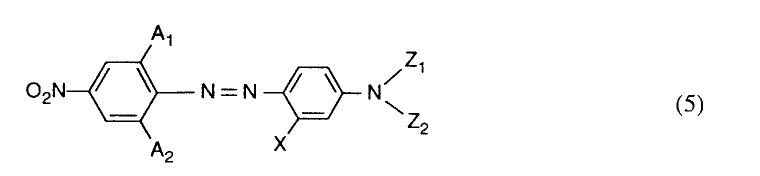 besonders bevorzugt, worin
A₁ Chlor, Brom, Cyan, Nitro, CF₃ oder Wasserstoff,
A₂ Wasserstoff, Chlor, Brom oder Cyan ,
X C₁-C₂-Alkyl, C₁-C₄-Alkylcarbonylamino, C₁-C₄-Alkoxycarbonylamino,
   C₁-C₄-Alkoxy-C₁-C₄-Alkylcarbonylamino oder
   C₁-C₄-Alkoxy-C₂-C₄-Alkoxycarbonylamino, und
Z₁ und Z₂ einen Rest der Formel 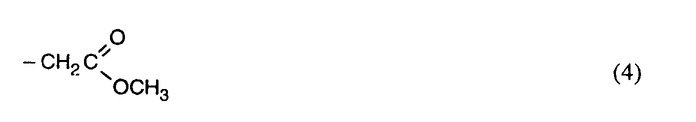
bedeuten.

Unter diesen sind diejenigen besonders bevorzugt, bei denen
A₁ Chlor, Cyan oder Nitro,
A₂ Wasserstoff, Chlor, Brom oder Cyan,
X C₁-C₂-Alkyl, C₁-C₄-Alkylcarbonylamino, C₁-C₄-Alkoxycarbonylamino,
   C₁-C₄-Alkoxy-C₁-C₄-Alkylcarbonylamino oder
   C₁-C₄-Alkoxy-C₂-C₄-Alkoxycarbonylamino, und
Z₁ und Z₂ CH₂COOCH₃ bedeuten.

Die neuen Azofarbstoffe der Formel (1) können nach an sich bekannten Verfahren hergestellt werden. Man erhält sie beispielsweise, indem man eine Verbindung der Formel 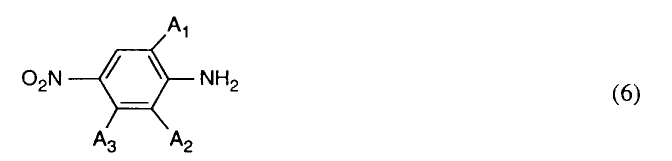 diazotiert und auf eine Kupplungskomponente der Formel 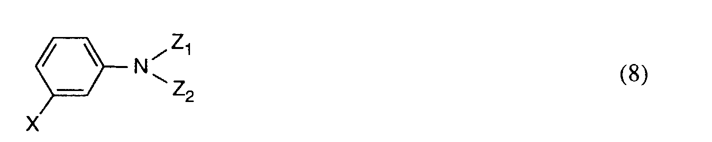 kuppelt, wobei A₁, A₂, A₃, X, Z₁ und Z₂ die unter der Formel (1) angegebenen Bedeutungen aufweisen.

Die Diazotierung der Verbindungen der Formel (6) erfolgt in an sich bekannter Weise, z.B. mit Natriumnitrit in saurem, z.B. salzsaurem oder schwefelsaurem, wässrigem Medium. Die Diazotierung kann aber auch mit anderen Diazotierungsmitteln, z.B. mit Nitrosylschwefelsäure ausgeführt werden. Bei der Diazotierung kann eine zusätzliche Säure im Reaktionsmedium anwesend sein, z.B. Phosphorsäure, Schwefelsäure, Essigsäure, Propionsäure, Salzsäure oder Mischungen dieser Säuren, z.B. Mischungen aus Phosphorsäure und Essigsäure. Zweckmässig wird die Diazotierung bei Temperaturen von -10 bis 30°C, z.B. von -10°C bis Raumtemperatur, durchgeführt.

Die Kupplung der diazotierten Verbindung der Formel (6) auf die Kupplungskomponente der Formel (8) erfolgt ebenfalls in bekannter Weise, beispielsweise in saurem, wässrigem oder wässrig-organischem Medium, vorteilhaft bei Temperaturen von -10 bis 30°C, insbesondere unter 10°C. Als Säuren verwendet man z.B. Salzsäure, Essigsäure, Schwefelsäure oder Phosphorsäure. Diazotierung und Kupplung können beispielsweise im Eintopfverfahren, also im gleichen Reaktionsmedium durchgeführt werden.

Die Diazokomponenten der Formeln (6) sind bekannt oder können auf an sich bekannte Art und Weise hergestellt werden.

Die Kupplungskomponenten der Formel (8) sind zum Teil ebenfalls bekannt oder können auf an sich bekannte Art und Weise hergestellt werden.

Die erfindungsgemässen Verbindungen der Formel (1) können als Farbstoffe zum Färben und Bedrucken von halbsynthetischen und insbesondere synthetischen hydrophoben Fasermaterialien, vor allem Textilmaterialien, verwendet werden. Textilmaterialien aus Mischgeweben, die derartige halbsynthetische bzw. synthetische hydrophobe Textilmaterialien enthalten, können ebenfalls mit Hilfe der erfindungsgemässen Verbindungen gefärbt oder bedruckt werden.

Als halbsynthetische Textilmaterialien kommen vor allem Cellulose 2½-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-cyclohexan; aus Polycarbonaten, z.B. solchen aus α,α-Dimethyl-4,4'-dihydroxy-diphenylmethan und Phosgen, aus Fasern auf Polyvinylchlorid- sowie Polyamid-Basis.

Die Applikation der erfindungsgemässen Verbindungen auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nicht-ionischen Dispergiermitteln und gegebenenfalls üblichen Quellmitteln (Carrier) bei Temperaturen zwischen 80 und 140°C. Cellulose-2½-acetat färbt man vorzugsweise zwischen ungefähr 65 bis 85°C und Cellulosetriacetat bei Temperaturen bis zu 115°C.

Die neuen Farbstoffe färben im Färbebad gleichzeitig anwesende Wolle und Baumwolle nicht oder nur wenig an (sehr gute Reserve), so dass sie auch gut zum Färben von Polyester/Wolle- und Polyester/Cellulosefaser-Mischgeweben verwendet werden können.

Die erfindungsgemässen Farbstoffe eignen sich zum Färben nach dem Thermosol-Verfahren, im Ausziehverfahren und für Druckverfahren.

Das genannte Textilmaterial kann dabei in den verschiedenen Verarbeitungsformen vorliegen, wie z.B. als Faser, Faden oder Vlies, als Gewebe oder Gewirke.

Es ist vorteilhaft, die erfindungsgemässen Farbstoffe vor ihrer Verwendung in ein Farbstoffpräparat zu überführen. Hierzu wird der Farbstoff vermahlen, so dass seine Teilchengrösse im Mittel zwischen 0,1 und 10 Mikron beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser Druckpasten und Färbebäder herstellen.

Beim Bedrucken wird man die üblichen Verdickungsmittel verwenden, z.B. modifizierte oder nichtmodifizierte natürliche Produkte, beispielsweise Alginate, British-Gummi, Gummi arabicum, Kristallgummi, Johannisbrotkernmehl, Tragant, Carboxymethylcellulose, Hydroxyethylcellulose, Stärke oder synthetische Produkte, beispielsweise Polyacrylamide, Polyacrylsäure oder deren Copolymere oder Polyvinylalkohole.

Die erfindungsgemässen Farbstoffe verleihen den genannten Materialien, vor allem dem Polyestermaterial, egale orange und rote bis blaue Farbtöne von sehr guten Gebrauchs-Echtheiten, wie vor allem guter Lichtechtheit, Thermofixier-, Plissier-, Chlor- und Nassechtheit wie Wasser-, Schweiss- und Waschechtheit; die Ausfärbungen sind ferner gekennzeichnet durch sehr gute Reibechtheit. Besonders hervorzuheben ist die gute Thermomigrationsechtheit der erhaltenen Färbungen.

Die erfindungsgemässen Farbstoffe können auch gut verwendet werden zur Herstellung von Mischnuancen zusammen mit anderen Farbstoffen. Selbstverständlich können auch Mischungen der erfindungsgemässen Farbstoffe untereinander verwendet werden.

Ausserdem eignen sich die erfindungsgemässen Farbstoffe auch gut zum Färben von hydrophobem Textilmaterial aus überkritischem CO₂.

Die vorstehend genannte Verwendung der erfindungsgemässen Azoverbindungen der Formel (1) stellt ebenso einen Gegenstand der vorliegenden Erfindung dar wie ein Verfahren zum Färben oder Bedrucken von halb-synthetischem oder synthetischem hydrophobem Fasermaterial, insbesondere Textilmaterial, das darin besteht, eine oder mehrere Verbindungen der Formel (1) auf das genannte Material aufzubringen oder es in dieses einzuarbeiten. Das genannte hydrophobe Fasermaterial ist vorzugsweise textiles Polyestermaterial. Weitere Substrate, die durch das erfindungsgemässe Verfahren behandelt werden können, sowie bevorzugte Verfahrensbedingungen sind vorstehend bei der näheren Erläuterung der Verwendung der erfindungsgemässen Verbindungen zu finden.

Ein weiterer Gegenstand der Erfindung ist das durch das genannte Verfahren gefärbte bzw. bedruckte hydrophobe Fasermaterial, vorzugsweise Polyester-Textilmaterial.

Die erfindungsgemässen Farbstoffe der Formel (1) eignen sich ausserdem für moderne Aufzeichnungsverfahren, wie z.B. Thermotransfer-Printing.

Die folgenden Beispiele veranschaulichen die Erfindung weiter, ohne sie darauf zu beschränken. Teile und Prozente beziehen sich, sofern nicht anders angegeben, auf das Gewicht.

Beispiel 1: 10,7 g m-Toluidin, 40 ml Bromessigsäuremethylester, 60 ml Methanol und 23,3 g Natriumcarbonat werden während 8 Stunden bei Rückflusstemperatur gerührt. Danach wird auf Raumtemperatur abgekühlt und abfiltriert. Das Filtrat wird am Rotationsverdampfer eingeengt und anschliessend destilliert. Bei 136 - 153° C bei 2 mbar erhält man 31 g eines gelben, zähflüssigen Oels der Formel 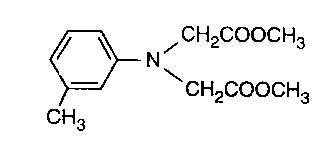 3,3 g 2-Cyan-4-nitroanilin werden auf übliche Art und Weise in konzentrierter Schwefelsäure diazotiert und bei einem pH-Wert zwischen 3 und 5 auf eine äquivalente Menge der vorstehend beschriebenen Kupplungskomponente gekuppelt. Das durch Filtration erhaltene Rohprodukt wird mit 100 ml Methanol versetzt, 2 Stunden bei Rückflusstemperatur gerührt und heiss abfiltriert. Dieser Reinigungsschritt wird wiederholt. Nach dem Trocknen erhält man 6,1 g Kristalle vom Schmelzpunkt 192 - 205° C. Der Farbstoff hat die Formel

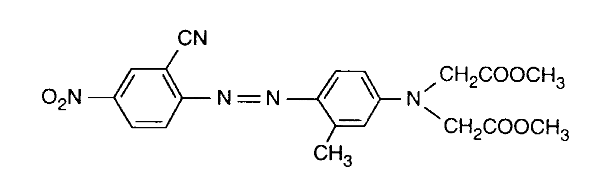

Er färbt Textilmaterial aus Polyester in roten Farbtönen. Die Färbungen weisen gute Echtheiten, insbesondere eine gute Thermomigrationsechtheit auf.

Beispiele 2 bis 35: Nach der in Beispiel 1 beschriebenen Arbeitsweise lassen sich auch die folgenden Farbstoffe herstellen. Sie färben Textilmaterial aus Polyester in den in der vorletzten Spalte der Tabelle 1 verzeichneten Nuancen.

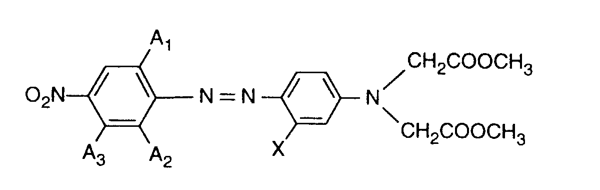

**Tabelle 1**

| Bsp. | A₁ | A₂ | A₃ | X | Nuance | λₘₐₓ(EtOH) |
|---|---|---|---|---|---|---|
| 2 | Cl | H | H | CH₃ | Braun | 458 nm |
| 3 | NO₂ | Br | H | CH₃ | Bordeaux | 488 nm |
| 4 | NO₂ | H | H | NHCOCH₃ | Rot | 500 nm |
| 5 | NO₂ | H | H | NHCOCH₂CH(CH₃)₂ | Rot | 510 nm |
| 6 | NO₂ | H | H | NHCOC₂H₅ | Rot | 502 nm |
| 7 | NO₂ | H | H | NHCOOC₂H₅ | Rot | 502 nm |
| 8 | CN | H | H | NHCOCH₃ | Rot | 508 nm |
| 9 | CN | H | H | NHCOOC₂H₅ | Rot | 506 nm |
| 10 | CN | H | H | NHCOC₂H₅ | Rot | 508 nm |
| 11 | Cl | H | H | NHCOCH₃ | Scharlach | 486 nm |
| 12 | CF₃ | H | H | NHCOCH₃ | Scharlach | 486 nm |
| 13 | NO₂ | CN | H | NHCOC₂H₅ | Violett | 558 nm |
| 14 | Cl | H | H | NHCONH₂ | Rot | 500 nm |
| 15 | NO₂ | H | H | CH₃ | Scharlach | 480 nm |
| 16 | CN | H | H | CF₃ | Orange | 470 nm |
| 17 | CN | Br | H | NHCOCH₃ | Violett | 542 nm |
| 18 | Cl | H | H | NHCOC₂H₅ | Scharlach | 486 nm |
| 19 | H | H | H | NHCOCH₂OCH₃ | Orange | 464 nm |
| 20 | CN | Cl | H | CH₃ | Bordeaux | 500 nm |
| 21 | SO₂CH₃ | Br | H | NHCOC₂H₅ | Rotbraun | |
| 22 | NO₂ | H | H | NHCOOCH₃ | Rot | 502 nm |
| 23 | H | H | H | NHCOCH₃ | Orange | 464 nm |
| 24 | H | H | H | NHCOC₂H₅ | Orange | 468 nm |
| 25 | NO₂ | Cl | H | NHCOCH₃ | Rubin | 518 nm |
| 26 | CN | H | H | NHCOOCH₃ | Rot | 506 nm |
| 27 | NO₂ | H | Cl | NHCOCH₃ | Rubin | |
| 28 | Br | Br | H | NHCOCH₃ | Braun | |
| 29 | SO₂CH₃ | H | H | NHCOOC₂H₅ | Braun | |
| 30 | CF₃ | Br | H | CH₃ | Braun | |
| 31 | NO₂ | H | Cl | CH₃ | Rubin | |
| 32 | CN | H | H | NHCOOC₂H₄OCH₃ | Rot | 506 nm |
| 33 | Cl | H | H | NHCOCH₂OCH₃ | Rot | 484 nm |
| 34 | CN | CN | H | NHCOCH₃ | Violett | 568 nm |
| 35 | CN | H | H | NHCOC₂H₄OC₂H₅ | Rot | 508 nm |

Beispiel 36: Kuppelt man das Diazoniumsalz von 2,5-Dichlor-4-nitroanilin nach der im Beispiel 1 beschriebenen Arbeitsweise auf die im Beispiel 38 beschriebene Kupplungskomponente so erhält man den Farbstoff der Formel 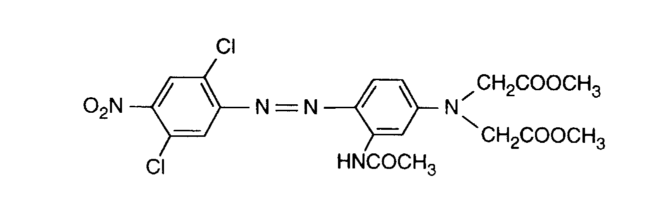 Dieser färbt Textilmaterial aus Polyester in scharlachroten Farbtönen. Die Färbungen weisen gute Echtheiten, insbesondere eine gute Thermomigrationsechtheit auf.

Beispiel 37: 1 g des im Beispiel 1 beschriebenen Farbstoffs wird zusammen mit 17 g Wasser und 2 g eines handelsüblichen Dispergators vom Typ Dinaphthylmethandisulfonat in einer Sandmühle gemahlen und in eine 5%ige Dispersion überführt.

Mit dieser Formulierung wird im Ausziehverfahren bei 130° C eine 0,5 %ige Färbung (bezogen auf Pigment und Substrat) auf Polyestergewebe erstellt und reduktiv gereinigt. Die so erzielte rote Färbung weist sehr gute Gebrauchsechtheiten und insbesondere eine ausgezeichnete Thermomigrationsechtheit auf.

Ebenfalls sehr gute Echtheiten lassen sich erzielen, wenn mit der genannten Formulierung ein Mischgewebe aus Polyester/Baumwolle (67 : 33) im Thermosolverfahren gefärbt wird (10 g/l Farbstoff, Flottenaufnahme 50 %, Fixiertemperatur 210° C ).

Zur Prüfung der Thermomigrationsechtheit wird das gefärbte Gewebe mit einem Textilweichmacher vom Typ Distearyl-diethylentriamin ausgerüstet und anschliessend während 30 Sekunden auf 180° C erhitzt. Danach werden Reibechtheit und Waschechtheit (60°C) der Probe geprüft.

Beispiel 38: 50 g 3-Aminoacetanilid, 180 g Chloressigsäuremethylester, 48 g Natriumcarbonat und 6 g Natriumbromid werden unter CO₂-Entwicklung auf 115° C erwärmt und während 10 Stunden bei dieser Temperatur gerührt. Das entstehende Wasser wird aus dem Reaktionsgefäss laufend als azeotropes Gemisch (Siedepunkt 95° C) mit Chloressigsäuremethylester abdestilliert. Nach dem Abkühlen wird die organische Phase mit 250 ml Wasser gewaschen und anschliessend unter reduziertem Druck destilliert. Als Rückstand erhält man 98 g braunes Oel der Verbindung der Formel 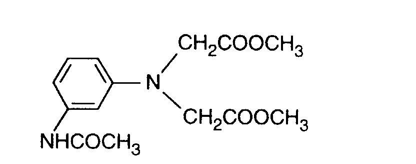 welches beim Abkühlen auf die Raumtemperatur fest wird.

Auf analoge Weise wie im Beispiel 38 beschrieben werden folgende in der Tabelle 4 aufgeführte Produkte der Formel 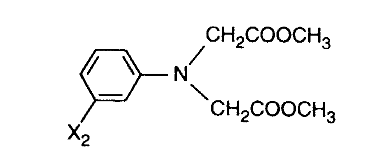 hergestellt.

**Tabelle 4**

| X₂ | Reaktionsdauer | Aspekt des Rohproduktes bei Raumtemperatur | Smp. (CH₃OH) |
|---|---|---|---|
| NHCOOC₂H₅ | 15 h | Oel | |
| NHCOC₂H₅ | 10 h | kristallin, | 104-110°C |
| NHCOCH₂OCH₃ | 15 h | kristallin | 96-100°C |

Beispiel 39: 50 g 3-Aminoacetanilid, 180 g Chloressigsäuremethylester, 34 g Lithiumcarbonat und 5 g Lithiumbromid werden unter CO₂-Entwicklung auf 115° C erwärmt und während 8 Stunden bei dieser Temperatur gerührt. Das entstehende Wasser wird aus dem Reaktionsgefäss laufend als azeotropes Gemisch (Siedepunkt 95° C) mit Chloressigsäuremethylester abdestilliert. Nach dem Abkühlen wird die organische Phase mit 250 ml Wasser gewaschen und anschliessend unter reduziertem Druck destilliert. Als Rückstand erhält man 87 g braunes Oel der Verbindung der Formel 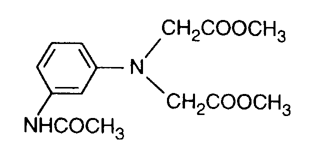 welches beim Abkühlen auf die Raumtemperatur fest wird.

Beispiel 40: 50 g 3-Aminoacetanilid, 180 g Chloressigsäuremethylester, 62 g Kaliumcarbonat und 7 g Kaliumbromid werden unter CO₂-Entwicklung auf 115° C erwärmt und während 8 Stunden bei dieser Temperatur gerührt. Das entstehende Wasser wird aus dem Reaktionsgefäss laufend als azeotropes Gemisch (Siedepunkt 95° C) mit Chloressigsäuremethylester abdestilliert. Nach dem Abkühlen wird die organische Phase mit 250 ml Wasser gewaschen und anschliessend unter reduziertem Druck destilliert. Als Rückstand erhält man ein braunes Oel der Verbindung der Formel 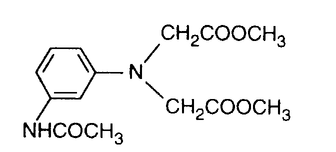 welches beim Abkühlen auf die Raumtemperatur fest wird.

Beispiel 41: 50 g 3-Aminoacetanilid, 180 g Chloressigsäuremethylester, 48 g Natriumcarbonat und 9 g Bromessigsäuremethylester werden unter CO₂-Entwicklung auf 115° C erwärmt und während 10 Stunden bei dieser Temperatur gerührt. Das entstehende Wasser wird aus dem Reaktionsgefäss laufend als azeotropes Gemisch (Siedepunkt 95° C) mit Chloressigsäuremethylester abdestilliert. Nach dem Abkühlen wird die organische Phase mit 250 ml Wasser gewaschen und anschliessend unter reduziertem Druck destilliert. Als Rückstand erhält man ein braunes Oel der Verbindung der Formel 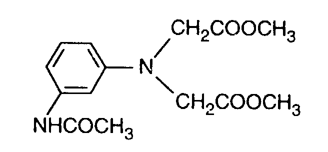 welches beim Abkühlen auf die Raumtemperatur fest wird.

Beispiel 42: 50 g 3-Aminoacetanilid, 180 g Chloressigsäuremethylester, 48 g Natriumcarbonat und 1 g Kaliumiodid werden unter CO₂-Entwicklung auf 100° C erwärmt und während 8 Stunden bei dieser Temperatur gerührt. Das entstehende Wasser wird aus dem Reaktionsgefäss laufend als azeotropes Gemisch (Siedepunkt 95° C) mit Chloressigsäuremethylester abdestilliert. Nach dem Abkühlen wird die organische Phase mit 250 ml Wasser gewaschen und anschliessend unter reduziertem Druck destilliert. Als Rückstand erhält man ein braunes Oel der Verbindung der Formel 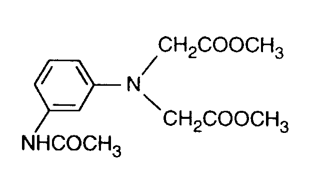 welches beim Abkühlen auf die Raumtemperatur fest wird.

## Patentansprüche

1. Farbstoffe der Formel 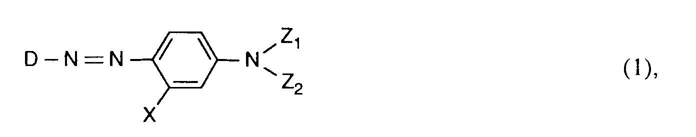 worin
D den Rest einer Diazokomponente der Formel 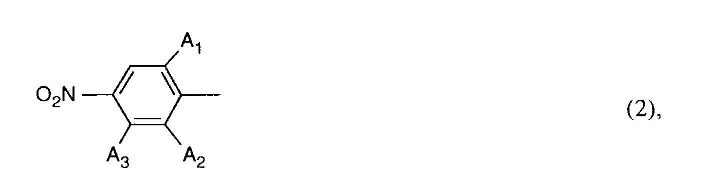 Z₁ und Z₂ einen Rest der Formel 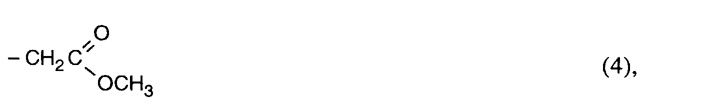
X C₁-C₂-Alkyl, C₁-C₄-Alkylcarbonylamino, C₁-C₄-Alkoxycarbonylamino,
C₁-C₄-Alkoxy-C₁-C₄-Alkylcarbonylamino oder
C₁-C₄-Alkoxy-C₂-C₄-Alkoxycarbonylamino,
A₁ Wasserstoff, Halogen, SO₂R₃, CF₃, NO₂ oder CN,
A₂ Wasserstoff, Halogen oder CN,
A₃ Wasserstoff oder Halogen und
R₃ C₁-C₆-Alkyl sind,
bedeuten.

2. Farbstoffe gemäss Anspruch 1, worin A₁ Chlor, Brom, Cyan, Nitro, CF₃ oder Wasserstoff bedeutet.

3. Farbstoffe gemäss einem der Ansprüche 1 und 2, worin A₂ Wasserstoff, Chlor, Brom oder Cyan bedeutet.

4. Farbstoffe gemäss einem der Ansprüche 1 bis 3, worin A₃ Wasserstoff bedeutet.

5. Farbstoffe gemäss Anspruch 1, worin A₁ Chlor, Cyan oder Nitro, A₂ Wasserstoff, Chlor, Brom oder Cyan und A₃ Wasserstoff bedeutet.

6. Farbstoffe der Formel 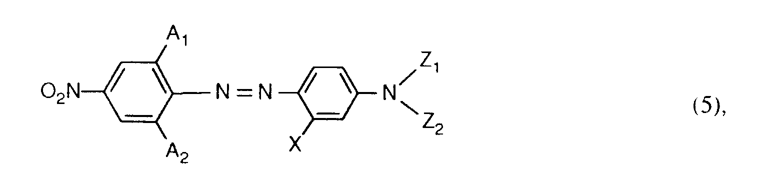 worin
A₁ Chlor, Brom, Cyan, Nitro, CF₃ oder Wasserstoff,
A₂ Wasserstoff, Chlor, Brom oder Cyan ,
X C₁-C₂-Alkyl, C₁-C₄-Alkylcarbonylamino, C₁-C₄-Alkoxycarbonylamino,
C₁-C₄-Alkoxy-C₁-C₄-Alkylcarbonylamino oder
C₁-C₄-Alkoxy-C₂-C₄-Alkoxycarbonylamino, und
Z₁ und Z₂ einen Rest der Formel 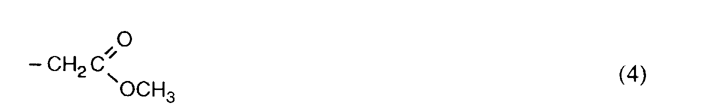
bedeutet.

7. Farbstoffe gemäss Anspruch 6, worin A₁ Chlor, Cyan oder Nitro bedeutet.

8. Verfahren zur Herstellung von Farbstoffen der Formel (1) gemäss Anspruch 1, dadurch gekennzeichnet, dass man eine Verbindung der Formel 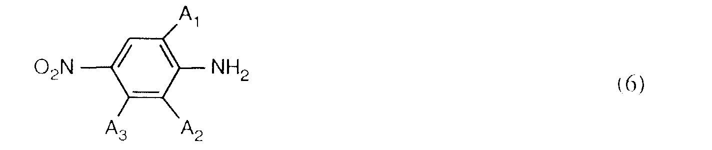 diazotiert und auf eine Kupplungskomponente der Formel 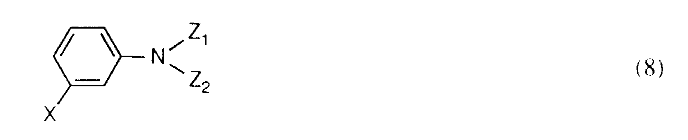 kuppelt, wobei A₁, A₂, A₃, X, Z₁ und Z₂ die unter der Formel (1) angegebenen Bedeutungen aufweisen.

9. Verwendung der Farbstoffe gemäss Anspruch 1 zum Färben oder Bedrucken von halbsynthetischem oder synthetischem hydrophobem Fasermaterial, inbesondere Textilmaterial.

10. Verwendung nach Anspruch 9 zum Färben oder Bedrucken von Textilmaterial aus Polyesterfasern.

11. Verfahren zum Färben oder Bedrucken von halbsynthetischem oder synthetischem hydrophobem Fasermaterial, insbesondere Textilmaterial dadurch gekennzeichnet, dass man eine oder mehrere der im Anspruch 1 definierten Verbindungen auf das genannte Material aufbringt oder diesem einverleibt.

12. Verfahren nach Anspruch 11, worin das hydrophobe Fasermaterial, vorzugsweise Textilmaterial, aus Polyesterfasern besteht.

13. Das gemäss Anspruch 11 oder 12 gefärbte oder bedruckte Material.

## Claims

1. A dye of formula 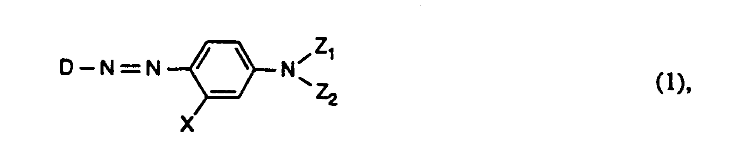 wherein
D is the radical of a diazo component of formula 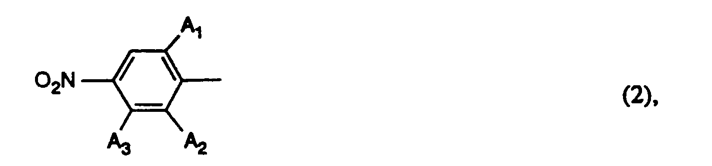 Z₁ and Z₂ are a radical of formula 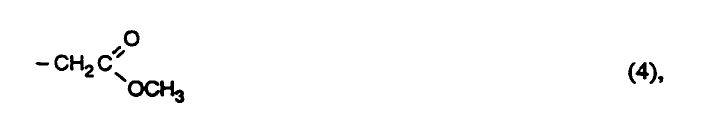
X is C₁-C₂alkyl, C₁-C₄alkylcarbonylamino, C₁-C₄alkoxy-carbonylamino, C₁-C₄alkoxy-C₁-C₄alkylcarbonylamino or C₁-C₄alkoxy-C₂-C₄alkoxycarbonylamino,
A₁ is hydrogen, halogen, SO₂R₃, CF₃, NO₂ or CN,
A₂ is hydrogen, halogen or CN,
A₃ is hydrogen or halogen, and
R₃ is C₁-C₆alkyl.

2. A dye according to claim 1, wherein A₁ is chloro, bromo, cyano, nitro, CF₃ or hydrogen.

3. A dye according to one of claims 1 and 2, wherein A₂ is hydrogen, chloro, bromo or cyano.

4. A dye according to one of claims 1 to 3, wherein A₃ is hydrogen.

5. A dye according to claim 1, wherein A₁ is chloro, cyano or nitro, A₂ is hydrogen, chloro, bromo or cyano and A₃ is hydrogen.

6. A dye of formula 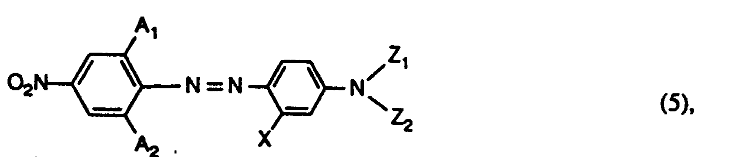 wherein
A₁ is chloro, bromo, cyano, nitro, CF₃ or hydrogen,
A₂ is hydrogen, chloro, bromo or cyano,
X is C₁-C₂alkyl, C₁-C₄alkylcarbonylamino, C₁-C₄alkoxy-carbonylamino, C₁-C₄alkoxy-C₁-C₄alkylcarbonylamino or C₁-C₄alkoxy-C₂-C₄alkoxycarbonylamino,
Z₁ and Z₂ are a radical of formula

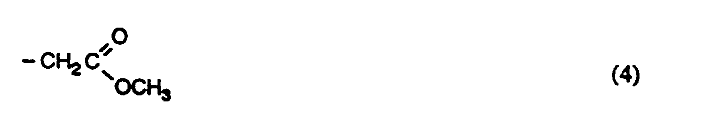

7. A dye according to claim 6, wherein A₁ is chloro, cyano or nitro.

8. A process for the preparation of a dye of formula (1) according to claim 1, which comprises diazotising a compound of formula 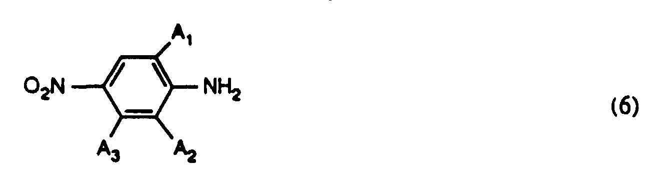 and coupling the diazonium salt of said compound to a coupling component of formula 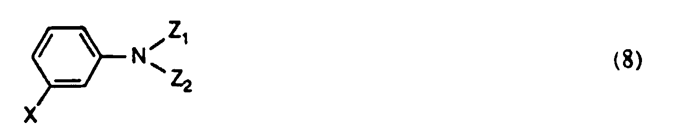 wherein A₁, A₂, A₃, A₄, X, Z₁ and Z₂ are as defined for formula (1).

9. The use of a dye according to claim 1 for dyeing or printing semi-synthetic or synthetic hydrophobic fibre material, especially textile material.

10. The use according to claim 9 for dyeing or printing textile material comprising polyester fibres.

11. A process for dyeing or printing semi-synthetic or synthetic hydrophobic material, especially textile material, which comprises applying to or incorporating in said material one or more than one compound as defined in claim 1.

12. A process according to claim 11, wherein the hydrophobic material, preferably textile material, consists of polyester fibres.

13. The material dyed or printed according to claim 11 or 12.

## Revendications

1. Colorants de formule 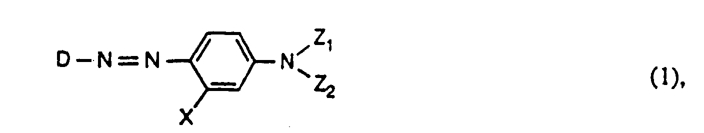 dans laquelle
D représente le résidu d'un composant diazoïque de formule 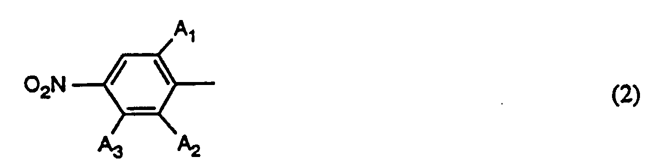 Z₁ et Z₂ un groupe de formule 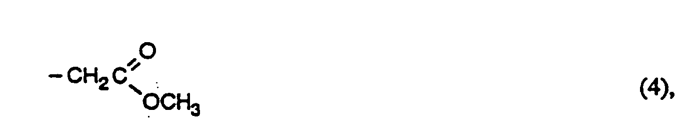
X représente un groupe alkyle en C₁-C₂, alkyle en C₁-C₄-carbonylamino, alcoxy en C₁-C₄-carbonylamino, alcoxy en C₁-C₄-alkyle en C₁-C₄-carbonylamino ou alcoxy en C₁-C₄-alcoxy en C₂-C₄-carbonylamino,
A₁ représente un atome d'hydrogène, d'halogène, un groupe SO₂R₃, CF₃, NO₂ ou CN,
A₂ représente un atome d'hydrogène, d'halogène ou CN,
A₃ est un atome d'hydrogène ou d'halogène, et
R₃ est un groupe alkyle en C₁-C₆.

2. Colorants selon la revendication 1, dans lesquels A₁ représente un atome de chlore, de brome, un groupe cyano, nitro, CF₃ ou un atome d'hydrogène.

3. Colorants selon une des revendications 1 ou 2, dans lesquels A₂ représente un atome d'hydrogène, un atome de chlore ou un groupe cyano.

4. Colorants selon une des revendications 1 à 3, dans lesquels A₃ représente un atome d'hydrogène.

5. Colorants selon la revendication 1, dans lesquels A₁ représente un atome de chlore, un groupe cyano ou nitro, A₂ représente un atome d'hydrogène, un atome de chlore, de brome ou un groupe cyano et A₃ représente un atome d'hydrogène.

6. Colorants de formule 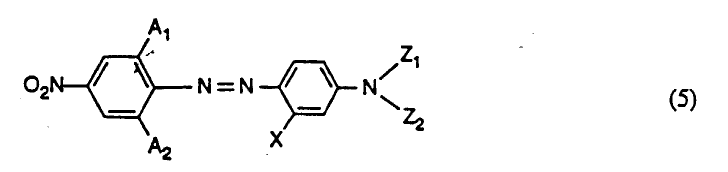 dans laquelle
A₁ représente un atome de chlore, de brome, un groupe cyano, nitro ou un atome d'hydrogène,
A₂ représente un atome d'hydrogène, un atome de chlore, de brome, ou un groupe cyano,
X représente un groupe alkyle en C₁-C₂, alkyle en C₁-C₄-carbonylamino, alcoxy en C₁-C₄-carbonylamino, alcoxy en C₁-C₄-alkyle en C₁-C₄-carbonylamino ou alcoxy en C₁-C₄-alcoxy en C₂-C₄-carbonylamino, et
Z₁ et Z₂ un groupe de formule

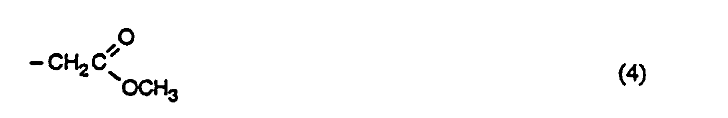

7. Colorants selon la revendication 6, dans lesquels A₁ représente un atome de chlore, un groupe cyano ou nitro.

8. Procédé de préparation de colorants de formule (1) selon la revendication 1, caractérisé en ce qu'on réalise la diazotation d'un composé de formule 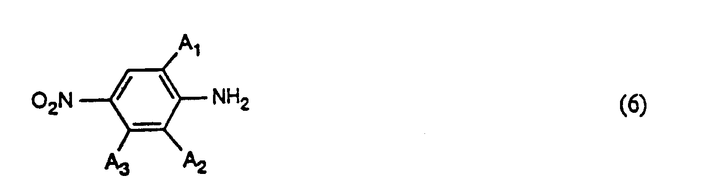 et la copulation avec un composant de copulation de formule 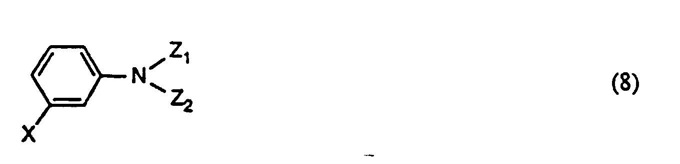 dans laquelle A₁, A₂, A₃, X, Z₁ et Z₂ présentent les significations indiquées sous la formule (1).

9. Utilisation des colorants selon la revendication 1 pour la teinture ou l'impression de matériau à base de fibres, hydrophobe semi-synthétique ou synthétique, en particulier de matériau textile.

10. Utilisation selon la revendication 9 pour la teinture ou l'impression de matériau textile de fibres de polyester.

11. Procédé pour la teinture ou l'impression de matériau à base de fibres, hydrophobe semi-synthétique ou synthétique, en particulier de matériau textile, caractérisé en ce qu'on dépose sur le matériau cité ou on incorpore dans celui-ci un ou plusieurs des composés définis dans la revendication 1.

12. Procédé selon la revendication 11, dans lequel le matériau à base de fibres hydrophobe, de préférence un matériau textile, est constitué de fibres de polyester.

13. Matériau teint ou imprimé selon la revendication 11 ou 12.
